# EUROPEAN PATENT SPECIFICATION

(11) **EP 4 209 141 B1**
(45) Date of publication and mention of the grant of the patent: **04.02.2026**
(21) Application number: 22215687.9
(22) Date of filing: 21.12.2022
(51) Int. Cl.: A41D 1/00, H01R 4/58, G06F 1/16, H01B 1/20, H05K 1/03, D03D 1/00

(54) **ZIPPER-TYPE CIRCUIT FABRIC AND SMART CLOTHES COMPOSED THEREOF**
REISSVERSCHLUSSARTIGES SCHALTUNGSGEWEBE UND DARAUS BESTEHENDE INTELLIGENTE KLEIDUNG
TISSU DE CIRCUIT DE TYPE FERMETURE À GLISSIÈRE ET VÊTEMENTS INTELLIGENTS COMPOSÉS DE CELUI-CI

(30) Priority: 06.01.2022 CN 202220026832 U; 18.11.2022 CN 202223075232 U
(43) Date of publication of application: 12.07.2023
(73) Proprietor: Lam, Tsz Kuen, San Po Kong, KLN (HK)
(72) Inventor: Lam, Tsz Kuen, San Po Kong, KLN (HK)
(74) Representative: Cabinet Chaillot

(56) References cited:
- US-A1- 2010 136 804
- US-A1- 2018 338 544
- US-B2- 9 966 697
- KLAMKA KONSTANTIN KLAMKA@ACM ORG ET AL: "Rapid Iron-On User Interfaces: Hands-on Fabrication of Interactive Textile Prototypes", PROCEEDINGS OF THE 2020 CHI CONFERENCE ON HUMAN FACTORS IN COMPUTING SYSTEMS, ACMPUB27, NEW YORK, NY, USA, 21 April 2020 (2020-04-21), pages 1 - 14, XP058461470, ISBN: 978-1-4503-6708-0, DOI: 10.1145/3313831.3376220

## Description

### TECHNICAL FIELD

The present invention relates to the technical field of smart wear, particularly to a zipper-type circuit fabric and smart clothes composed thereof.

### BACKGROUND ART

In the traditional garment industry, zipper as a cross-century invention has a history of more than 100 years. It is a component that temporarily joins two fabrics. The uses of zippers include: enlarging the opening to facilitate the passage of objects; joining separated fabrics; and decoration.

In the field of wearable devices such as artificial intelligent clothes, garments using one or more sensors that can sense biometric features have not been widely used. To some extent, it is because these garments may be restricted by the acceptable input types and versatility as well as by garment comfort and appearance. According to the latest market survey of wearable devices, more and more technology companies are cooperating with garment brands to develop smart clothing, and some even transfer some functions of smartphones to clothing to meet the needs of different scenarios and achieve direct operation on the human body instead of touching a touch screen with fingers. For example, Google cooperated with a clothing brand and introduced a smart jacket. The clothes are equipped with a touch sensor on a cuff, so that the user can play music, answer the phone and look at maps. This design ensures safe driving for users in a riding environment. Just as humans have shifted from using traditional personal computers to smartphones, the development of wearable devices will shift from stand-alone singularity to multifunctionality, energy efficiency, versatility, flexibility, and adaptability to different environments.

On the one hand, flexible sensors as the core technology of wearable devices can be used to manufacture devices on large substrates and create ultra-flexible perceptive systems that are thin and can be rolled up or folded without changing their functionality. According to the latest market research on flexible sensor technology, the U.S. flexible sensor market is expected to grow from $3.6 billion to $7.6 billion by 2027. The development of flexible material science lays a foundation for the development of smart materials and for the mass manufacturing of smart clothing.

On the other hand, in the field of smart wearable devices, human-machine remote interaction, remote operation, real-time transmission of physiological signals of human bodies, medical monitoring of human health, bionic robot control, human posture correction, sports training aids, artificial intelligence clothing design, etc. will be widely used in the sector of artificial intelligence. According to the prediction of analysts, compared with smart products such as bracelets and watches, smart clothes have stronger viscosity and are expected to become the next big thing for the development of wearable devices, driving the development of the smart wearable field.

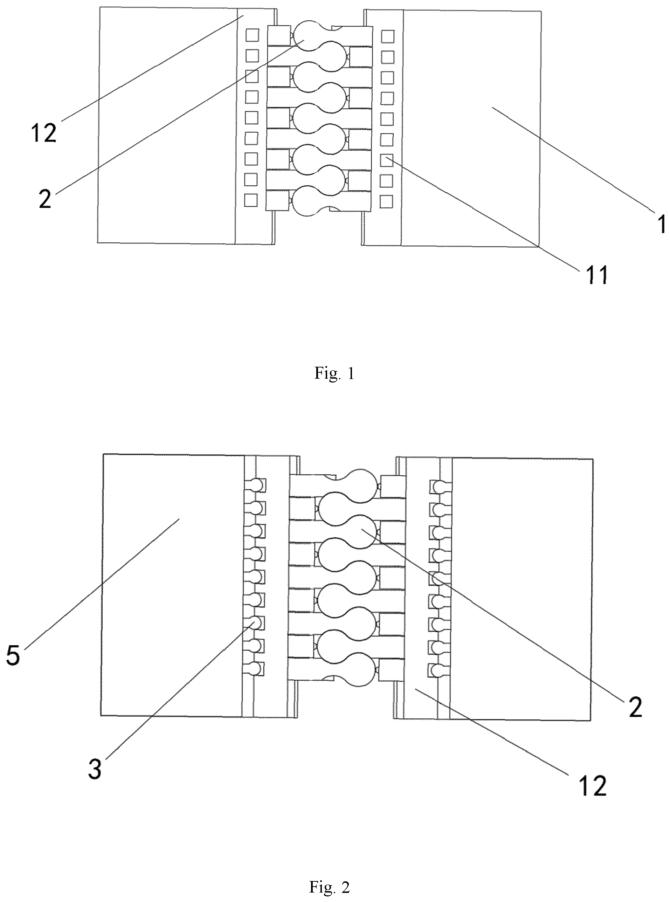

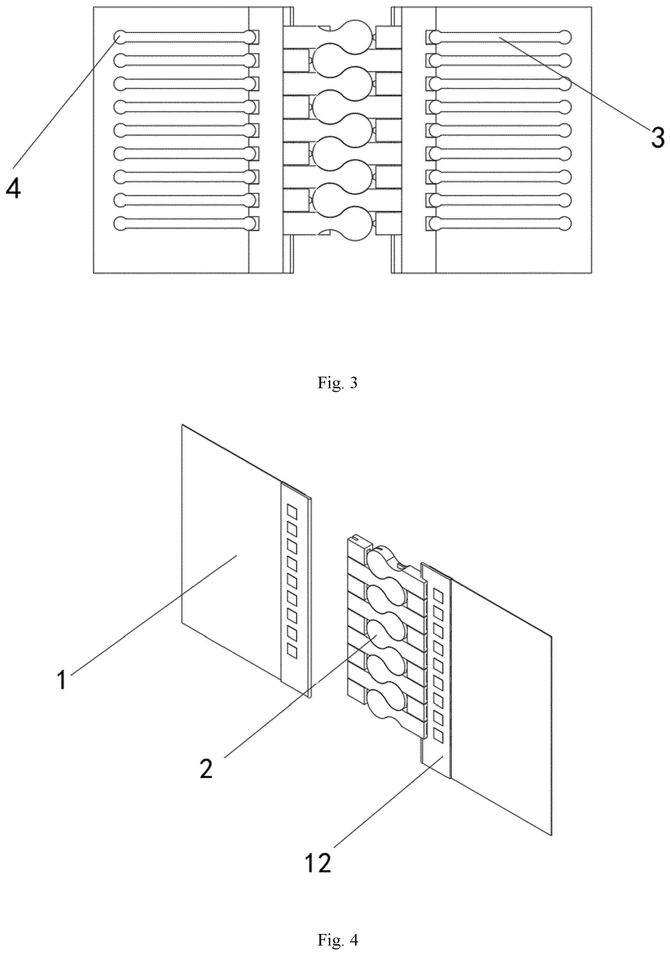

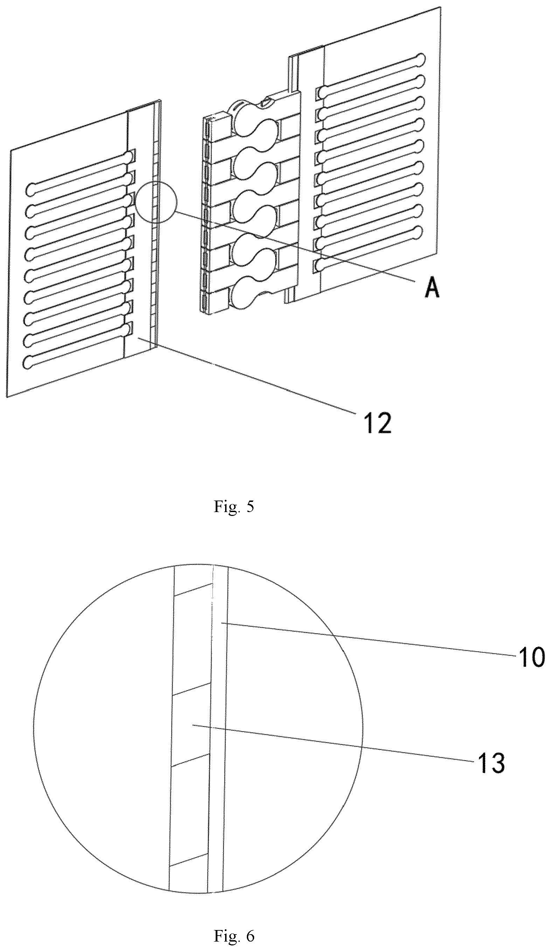

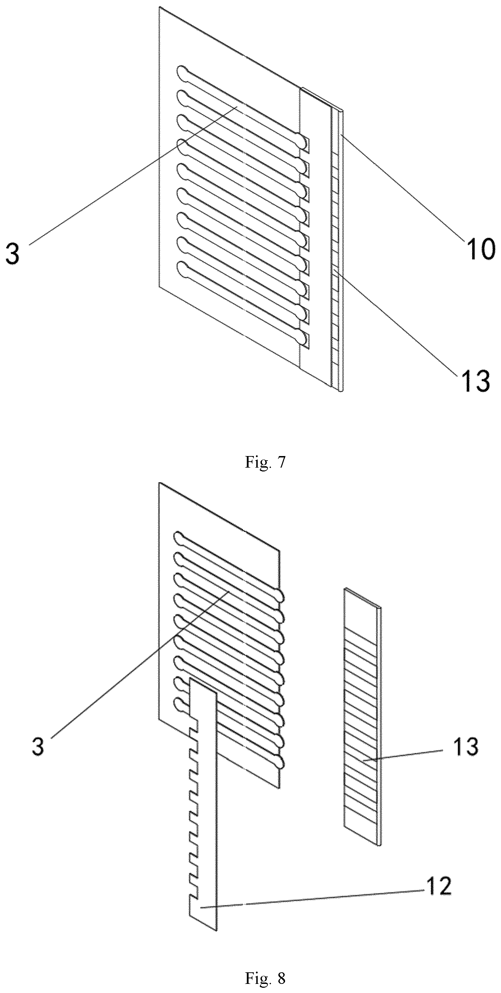

KLAMKA KONSTANTIN KLAMKA@ACM ORG ET AL, "Rapid Iron-On User Interfaces: Hands-on Fabrication of Interactive Textile Prototypes", PROCEEDINGS OF THE 2020 CHI CONFERENCE ON HUMAN FACTORS IN COMPUTING SYSTEMS, ACMPUB27, NEW YORK, NY, USA, (20200421), doi:10.1145/3313831.3376220, ISBN 978-1-4503-6708-0, pages 1 - 14, XP058461470 [X] 1 * page 4, column 1 - page 7, column 1; figures 1D, 4B, 6A, 6B, 8 * [A] 4-12 [I] 2,3, discloses the preamble of claim 1. It discloses rapid iron-on user interfaces for rapid prototyping of interactive textiles, which builds on heat-activated adhesive materials consisting of smart textiles and printed electronics, comprising a handheld dispenser tool for directly applying continuous functional tapes of desired length as well as discrete patches, allowing for creating complex circuits, utilizing commodity textile accessories and sketching custom-shaped I/O modules.

### SUMMARY OF THE INVENTION

An objective of the present invention is to overcome the defects of the prior art and provide a zipper-type circuit fabric and smart clothes composed thereof, which are internally provided with a plurality of conductive wires and flexible sensors that have certain ductility, are durable and waterproof and can be electrically connected to a plurality of electronic devices by means of a zipper body and perform recognition of body motion behavior.

The invention is as defined in claim 1.

The objective of the present invention is achieved through the following technical solution :
A zipper-type circuit fabric and smart clothes composed thereof, comprising a conductive fabric body, a zipper body, flexible capacitive sensors, flexible resistive sensors, a clothes body and glove bodies. A plurality of conductive wires and conductive contacts are distributed inside the conductive fabric body. The conductive wires can transmit electrical signals and achieve flow of data. The conductive contacts are arranged at both ends of the conductive wires. The distribution and shape of the conductive wires can be set according to the actual needs so that the conductive wires are distributed inside the conductive fabric body in different forms, such as: transverse, longitudinal, inclined, angle-folded and bent. A plurality of conductive contacts are arranged on two sides of the exterior of the zipper body. An end of the conductive fabric body is connected to one side of the zipper body. During use, after the zipper body is closed, electrical signals can be transmitted from one side of the zipper to the other side. The plurality of conductive wires and conductive contacts are electrically connected to a plurality of electronic components. The conductive fabric body can perform data exchange with external electronic components and is provided with an insulating fabric waterproof layer. During use, in order to avoid electric leakage and data loss, the plurality of conductive contacts and the plurality of conductive wires are covered by the insulating fabric waterproof layer. A stretchable adhesive-coated waterproof layer is arranged on each of the two sides of the conductive wires. People wearing clothes made of the zipper-type circuit fabric do not need to worry about disengagement or breakage of conductive wires caused by movements of human body. The conductive wires are internally provided with a first conductive medium. The first conductive medium includes rectangular elongated conductive media and folded conductive media, the elongated conductive media can improve the integrity of the conductive wires and the insulating fabric waterproof layer. When the zipper-type circuit fabric is stretched or deformed, the folded conductive media can be stretched or extended together. Alternatively, not being part of the claimed invention, the conductive contacts can be connected by means of a conductive fabric belt. The user may, as needed, connect the unconnected conductive wires by means of the conductive fabric belt. Not being part of the claimed invention, the upper layer of the conductive fabric belt is an insulating fabric, the middle is arranged a second conductive medium (not being part of the claimed subject-matter), the lower layer is a stretchable adhesive coating, and the second conductive medium is provided with a plurality of conductive nodes and includes rectangular elongated conductive media and folded conductive media. The second conductive medium (not being part of the claimed subject-matter) and the first conductive medium are the same in terms of the material forming method, except that the conductive fabric belt and the zipper-type circuit fabric are independent of each other. The flexible sensors are flexible capacitive sensors or flexible resistive sensors. Each of the flexible capacitive sensors consists of an upper electrode layer, a lower electrode layer and a middle dielectric layer sandwiched by the upper and lower electrode layers. The electrode layers are made of a plurality of conductive media. The middle dielectric layer is made of a non-conductive flexible organosilicon film material. Each of the flexible resistive sensors is composed of two adjacent conductive media and a graphite coated silicone layer with certain conductivity. The flexible sensors and the conductive fabric body form an integral body, of which surface is also provided with an insulating fabric waterproof layer to avoid electric leakage and data loss during use.

There are a plurality of the folded conductive media with a wavelength of L and an amplitude of M, and a rectangular elongated conductive medium with a length of 2M and a width of M is arranged on the central axis position at each interval of N sawtooth waves. The rectangular elongated conductive media can serve as conductive nodes and are connected to conductive fabric, or other conductive wires. When the zipper-type circuit fabric is bent or deformed, the folded conductive media can be bent or deformed together with the zipper-type circuit fabric.

The conductive wires are connected to the zipper body. One side of the first layer of the zipper body is a reinforcing core. When the zipper body is used, the reinforcing core can strengthen the firmness of the occlusion between the circuit fabric and the zipper teeth. The other side is a zipper fabric belt with a marking position. The second layer is a plurality of parallel striped conductive media with a width of K and perpendicular to the reinforcing core. The striped conductive media are electrically connected to the conductive wires. When the zipper body is closed, the zipper teeth are engaged with each other so that the conductive wires on different sides form electrical connection via the zipper teeth. The marking position facilitates connection of the conductive wires. The distance between the center points of two adjacent parallel striped conductive media is W, forming a clear boundary between conductive media. The third layer is an insulating layer covering the parallel striped conductive media. The insulating layer exposes a separate conductive medium with a width of K and the marking position, and is laminated with other conductive wires.

The zipper body is provided with a first tooth and a second tooth, which are distributed left and right in a stagger manner. When a closed circuit needs to be formed, the zipper head is pulled to bring the head of the first tooth into the second tooth and make the first teeth on the left and right engaged with each other, thereby closing the zipper body. The upper end of the first tooth is provided with a placement slot, the lower end is provided with a placement block, and the placement slots and placement blocks on different sides form a structure of left-right staggered buckling to effectively enhance vertical tensile strength and improve water resistance. When the zipper body is closed, the placement slots and placement blocks on the left first tooth and the right first tooth fit into each other. The middle of the second tooth is provided with a metal probe, the middle of the first tooth is provided with a connecting slot, and the metal probe and the connecting slot both are electrically connected to the parallel striped conductive media. After the zipper body is closed, by placing the metal probe into the connecting slot, the conductive wires on the left and right sides form a closed circuit by means of the zipper.

In an embodiment, outside of the second tooth is an elastic resin zipper tooth, the metal probe is a V-shaped metal clip, the rear end of the V-shaped metal clip has a first clamping portion, and the first clamping portion clamps the parallel striped conductive media. During use, the second tooth is placed on the zipper fabric belt and then squeezed so that the second tooth is clamped with the zipper fabric belt. At the same time, the first clamping portion is also squeezed, thereby clamping the parallel striped conductive media and forming an electrical connection. Outside of the first tooth is a rigid resin zipper tooth, the connecting slot is an H-shaped metal clip, the rear end of the H-shaped metal clip has a second clamping portion, and the second clamping portion clamps the parallel striped conductive media. During use, the first tooth is placed on the zipper fabric belt and then squeezed so that the first tooth is clamped with the zipper fabric belt. At the same time, the second clamping portion is also squeezed, thereby clamping the parallel striped conductive media and forming an electrical connection. When the zipper is fastened, the first tooth is closely engaged with the second tooth, and at the same time, the V-shaped metal clip is placed into and fit with the H-shaped metal clip to form a circuit.

In an embodiment, outside of the second tooth is an elastic resin zipper tooth, the metal probe is a V-shaped metal clip, the rear end of the V-shaped metal clip has a first clamping portion, and the first clamping portion clamps the parallel striped conductive media. During use, the second tooth is placed on the zipper fabric belt and then squeezed so that the second tooth is clamped with the zipper fabric belt. At the same time, the first clamping portion is also squeezed, thereby clamping the parallel striped conductive media and forming an electrical connection. Outside of the first tooth is a rigid resin zipper tooth, the inner connecting slot is a Y-shaped metal clip, the rear end of the Y-shaped metal clip has a third clamping portion, and the third clamping portion clamps the parallel striped conductive media. During use, the first tooth is placed on the zipper fabric belt and then squeezed so that the first tooth is clamped with the zipper fabric belt. At the same time, the third clamping portion is also squeezed, thereby clamping the parallel striped conductive media and forming an electrical connection. When the zipper is fastened, the first tooth is closely engaged with the second tooth, and at the same time, the V-shaped metal clip is placed into and fit with the Y-shaped metal clip to form a circuit.

In an embodiment, the first conductive medium is made of 316L stainless steel metal fiber sewing threads. Not being part of the claimed subject-matter, the second conductive medium is also made of 316L stainless steel metal fiber sewing threads. The first conductive medium forms rectangular elongated conductive media and folded conductive media by the zigzag stitching method, the surface threads of the first conductive medium is insulating surface yarn, and the bottom threads is stainless steel metal fiber sewing threads to improve the integrity of the first conductive medium and the insulating fabric waterproof layer. Not being part of the claimed subject-matter, the second conductive medium forms rectangular elongated conductive media and folded conductive media by the zigzag stitching method, the surface threads of the second conductive medium is insulating surface yarn, and the bottom threads is stainless steel metal fiber sewing threads to improve the integrity of the second conductive medium and the insulating fabric waterproof layer.

In an embodiment, the first conductive medium, and the second conductive medium in an embodiment not being part of the claimed subject-matter, are made of resilient conductive adhesive and directly laminated on the insulating fabric waterproof layer, the lamination method can be soldering, bonding or sewing, etc, the lower layer is a liftable adhesive coating, and the resilient conductive adhesive adopts 60-80% conductive particles, 20-30% adhesive, and 5-10% gelling agent. During production, the conductive adhesive is laminated directly to the bottom of the insulating fabric waterproof layer, forming elongated conductive media. The lower layer is a stretchable adhesive coating, which can cover the striped conductive media in a manner of strips at intervals to fix the conductive fabric belt to the material on the surface layer of the fabric.

In an embodiment, the smart clothes body is made through tailoring of the conductive fabric body and seam splicing by fabric cut piece. Detachable glove bodies are arranged at the cuffs of the smart clothes body. Zipper teeth on a side of a zipper body are arranged at each cuff of the clothes body and connected to the zipper teeth on the other side of the zipper body at the sleeve of a glove body. When the zipper body is closed, the conductive fabric body of the glove sleeve is connected to the conductive fabric body of the clothes cuff to achieve electrical connection between the glove and the clothes body. The zipper body at each clothes cuff can also be electrically connected to a flexible screen. The flexible screen establishes an electrical connection with the zipper teeth on the two sides of the zipper body by means of the conductive fabric body. A Velcro sticker is arranged on the back of the flexible screen. During use, the flexible screen is laminated with the external surface of the cuff, and then the zipper is pulled to close the zipper body.

In an embodiment, the front of the smart clothes body is provided with a zipper body. When the zipper body is closed, the conductive wires on the two sides of the zipper form a closed circuit. A plurality of flexible sensors are arranged at the joints of the smart clothes body and the glove bodies, are electrically connected to the conductive wires and can identify folding at the joints of the smart clothes or the gloves, thereby judging movements of the human body. A plurality of conductive contacts distributed inside pockets are electrically connected to external electronic devices, which can be a central processor unit (CPU), a 5G communication device or a feedback vibration module.

### Advantages of the present invention:

The present invention can satisfy the needs for power supply, stretching and waterproofing of electronic components in smart clothes, and uses conductive contacts to achieve connection of electronic components by means of conductive fabric body and zipper body. Flexible sensors form an integral body with the conductive fabric body, and can move freely in smart clothes and other wearable devices and convert various movements of human body into different electrical signals to identify and monitor human movements. Alternatively, a power source can be used to supply power in a centralized manner. For example, a portable power source is put in a zippered pocket and switched on or off by closing or opening the zipper body. In addition, as the zipper body is flexible, other electronic components can be added (or replaced) by expanding the interface of the zipper body connector to meet the individualized requirements for configuration of the circuit system. Further, not like wireless connection, wired connection is less likely to be interfered by surrounding electromagnetic environments, so it is a stable electrical connection.

### BRIEF DESCRIPTION OF THE DRAWINGS

In order to more clearly describe the technical solutions in the embodiments of the present invention, attached drawings that need to be used in the embodiments will be briefly described below. It should be understood that the following attached drawings only show some embodiments of the present invention, so they should not be deemed as a limitation to the scope. Those of ordinary skill in the art can also obtain other drawings without creative work based on these drawings.
Fig. 1 is structural schematic view 1 of the present invention;
Fig. 2 is structural schematic view 2 of the present invention;
Fig. 3 is structural schematic view 3 of the present invention;
Fig. 4 is structural schematic view 4 of the present invention;
Fig. 5 is structural schematic view 5 of the present invention;
Fig. 6 is an enlarged view of Area A in Fig. 5 of the present invention;
Fig. 7 is structural schematic view 1 of a zipper fabric belt provided by the present invention;
Fig. 8 is structural schematic view 2 of a zipper fabric belt provided by the present invention;
Fig. 9 is structural schematic view 3 of a zipper fabric belt provided by the present invention;
Fig. 10 is an enlarged view of Area B in Fig. 9 of the present invention;
Fig. 11 is structural schematic view 4 of a zipper fabric belt provided by the present invention;
Fig. 12 is an enlarged view of Area C in Fig. 11 of the present invention;
Fig. 13 is a structural schematic view of a conductive fabric body according the present invention and a conductive fabric belt which is not part of the claimed invention.
Fig. 14 is structural schematic view 1 of a zipper body provided by the present invention;
Fig. 15 is a structural schematic view of a second tooth provided by the present invention;
Fig. 16 is a structural schematic view of a first tooth provided by the present invention;
Fig. 17 is structural schematic view 2 of a zipper body provided by the present invention;
Fig. 18 is a structural schematic view of a V-shaped metal clip provided by the present invention;
Fig. 19 is a structural schematic view of an H-shaped metal clip provided by the present invention;
Fig. 20 is structural schematic view 3 of a zipper body provided by the present invention;
Fig. 21 is a structural schematic view of a Y-shaped metal clip provided by the present invention;
Fig. 22 is a structural schematic view of a clothes body provided by the present invention;
Fig. 23 is a schematic view of a deformable capacitor in a flexible capacitive sensor provided by the present invention;
Fig. 24 is a schematic view of the circuit of a flexible resistive sensor provided by the present invention;
Fig. 25 is a specific structural schematic view of a flexible capacitive sensor;
Fig. 26 is a specific structural schematic view of a flexible resistive sensor.

In the figures: conductive fabric body 1, zipper body 2, conductive wire 3, conductive contact 4, insulating fabric waterproof layer 5, first conductive medium 6, rectangular elongated conductive medium 61, folded conductive medium 62, conductive fabric belt 7, second conductive medium 8, conductive node 9, reinforcing core 10, marking position 11, zipper fabric belt 12, parallel striped conductive medium 13, first tooth 14, second tooth 15, placement slot 16, placement block 17, metal probe 18, connecting slot 19, V-shaped metal clip 20, first clamping portion 21, H-shaped metal clip 22, second clamping portion 23, Y-shaped metal clip 24, third clamping portion 25, clothes body 27, glove body 28, conductive electrode layer 29, flexible organosilicon film 30, graphite coated silicone layer 31, flexible capacitive sensor 32, and flexible resistive sensor 33.

### DETAILED DESCRIPTION

For easier understanding of the present invention, the present invention will be more comprehensively described below by referring to related attached drawings. The attached drawings show preferred embodiments of the present invention, but the present invention can be implemented in many different forms and are not limited to those described herein. On the contrary, the purpose of providing these implementation forms is for clearer and more comprehensive understanding of the content disclosed by the present invention.

It should be noted that when it is said that a component is "fixed to" another component, it can be directly on another component or there may be a component between them. When a component is considered to "be connected to" another component, it can be directly connected to another component or there may be a component between them at the same time. Terms "vertical", "horizontal", "left", "right" and similar expressions used herein is for the purpose of illustration only, and do not represent the only implementation form.

Unless otherwise defined, all technical and scientific terms used herein have the same meanings as those commonly understood by those skilled in the technical field of the present invention. The terms used in the description of the present invention herein are only for the purpose of describing specific embodiments and not intended to limit the present invention. The term "and/or" as used herein includes any and all combinations of one or more relevant listed items.

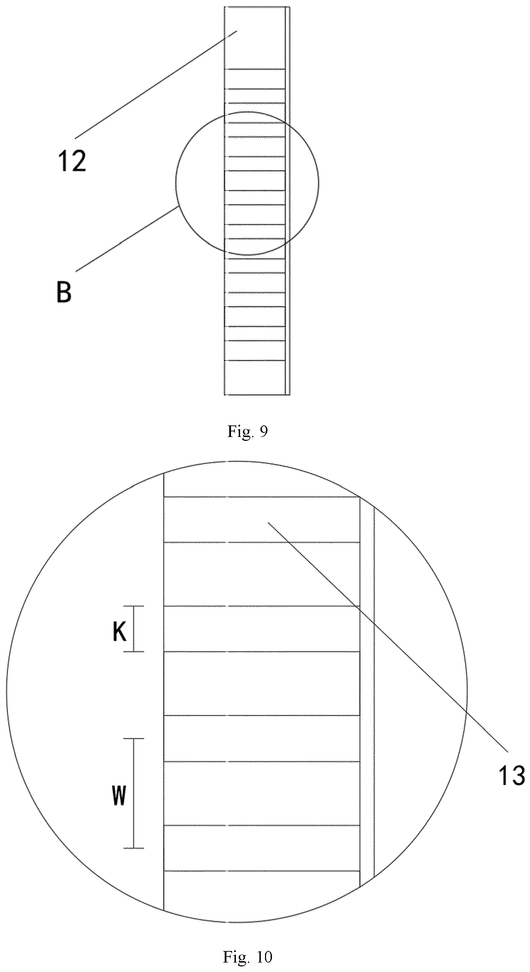

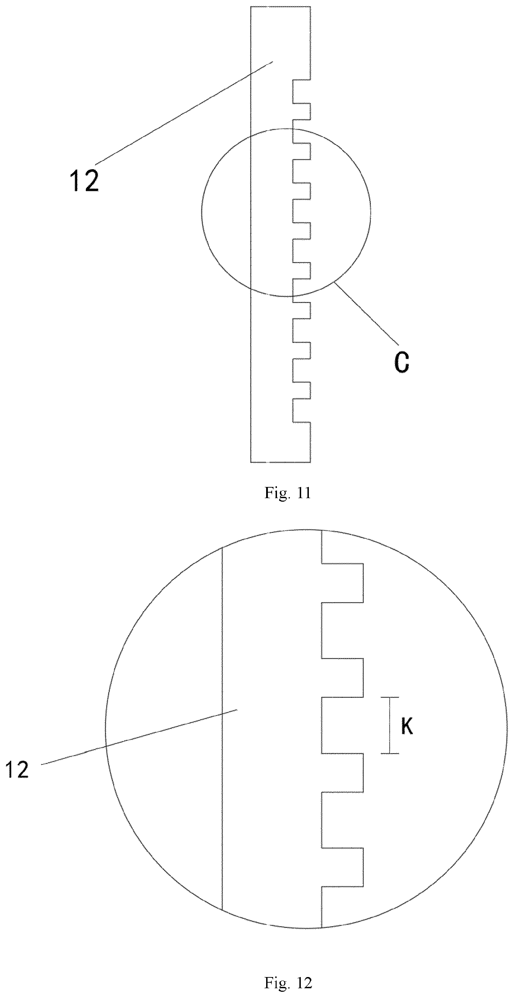

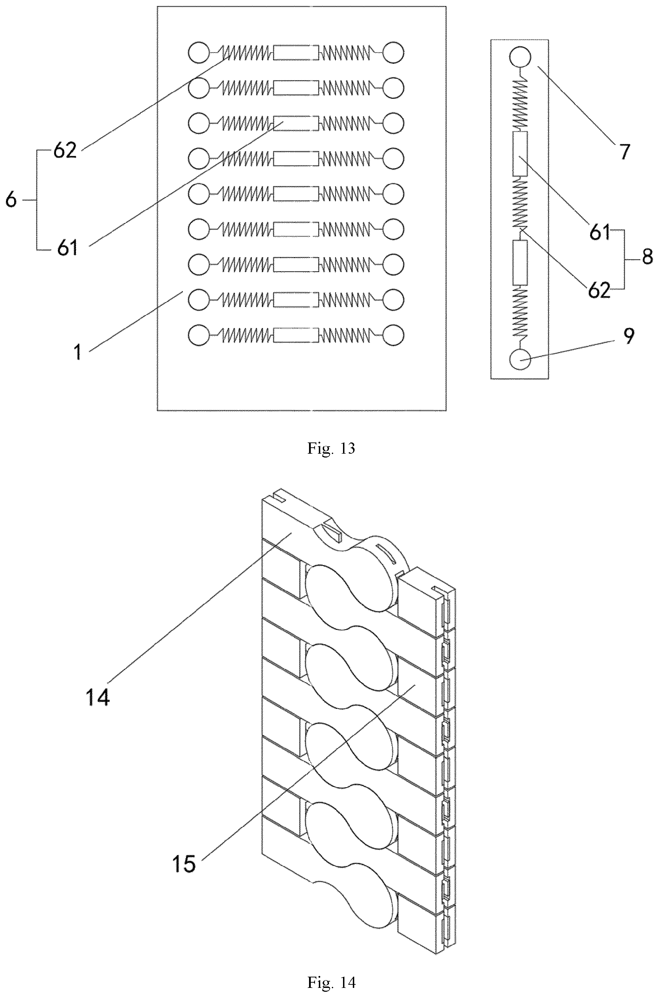

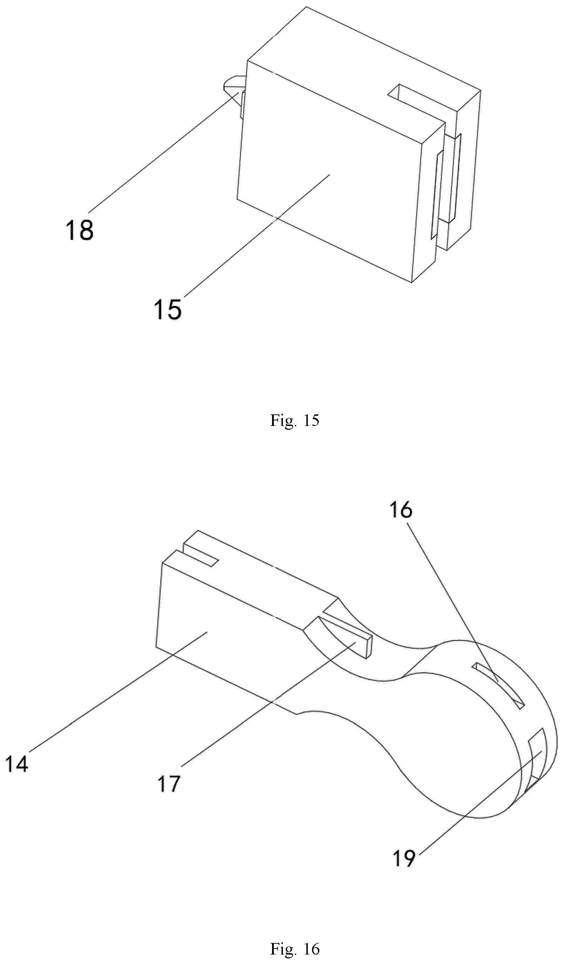

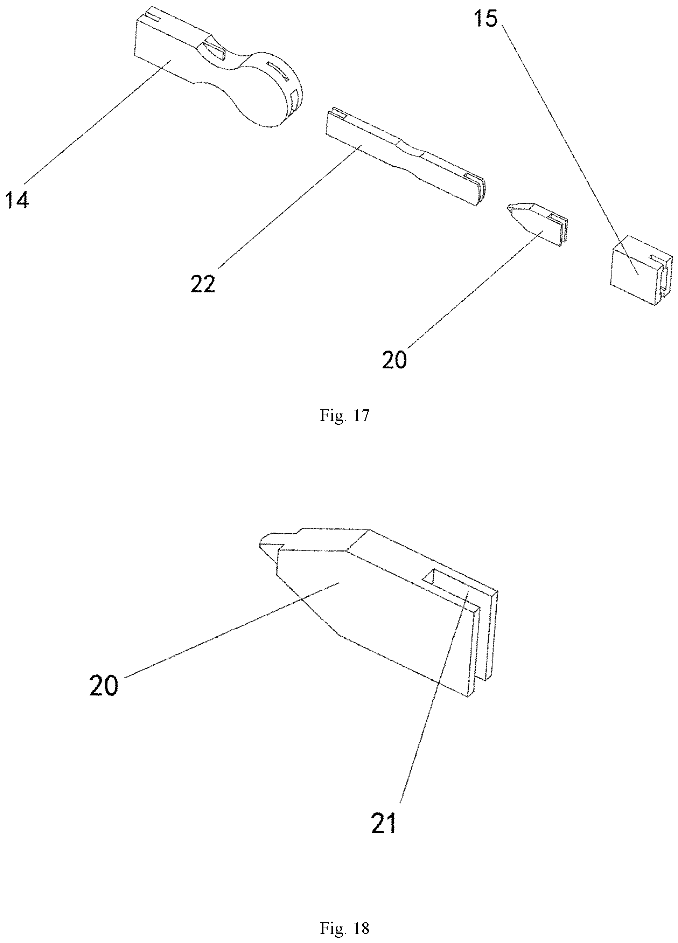

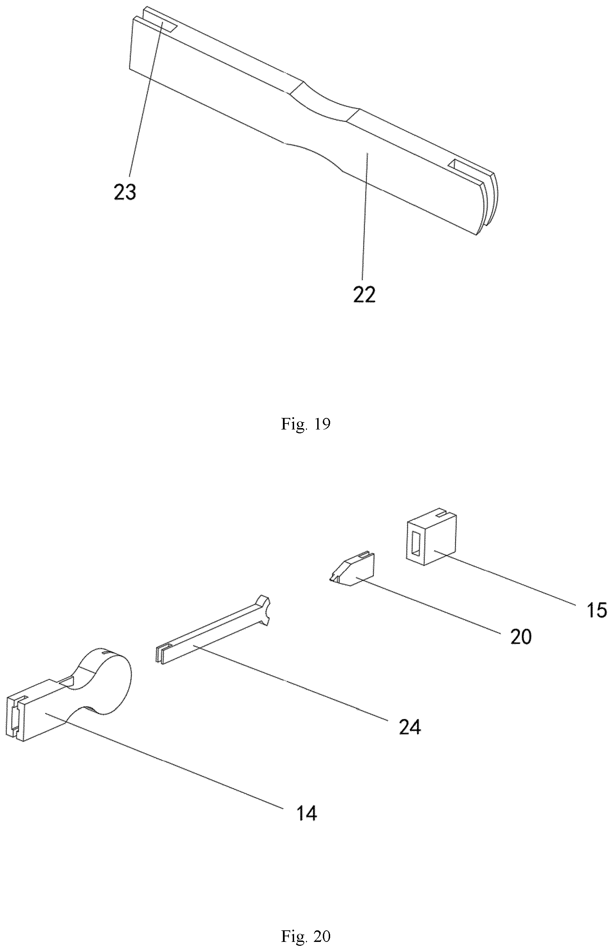

Please refer to Fig. 1 to Fig. 26: a zipper-type circuit fabric and smart clothes composed thereof, comprising a conductive fabric body 1, a zipper body 2, flexible capacitive sensors 32, flexible resistive sensors 33, a clothes body 27 and glove bodies 28. The zipper body 2 can be arranged on the two sides of the edges of the conductive fabric body 1 so that the conductive fabric body 1 forms a complete closed circuit. A reliable, flexible and convenient connection solution can be provided for the circuit integration system of rigid electronic components and flexible electronic components of artificial intelligent clothes and other wearable devices. A plurality of conductive wires 3 and conductive contacts 4 are distributed inside the conductive fabric body 1. The conductive contacts 4 are arranged on the conductive wires 3. To facilitate circuit design, a plurality of conductive contacts 4 are arranged on two sides of the exterior of the zipper body 2, and the zipper body 2 is electrically connected to the conductive contacts 4 so that the zipper body 2 has a power-on function. An end of the conductive fabric body 1 is connected to a side of the zipper body 2. The plurality of conductive wires 3 and conductive contacts 4 can be electrically connected to a plurality of electronic components to achieve various functions. The conductive fabric body 1 is provided with an insulating fabric waterproof layer 5 to play an effect of insulation and waterproofing and avoid electric leakage. The plurality of conductive contacts 4 and the plurality of conductive wires 3 are covered by the insulating fabric waterproof layer 5. A stretchable adhesive-coated waterproof layer is arranged on each of the two sides of the conductive wires 3. The conductive wires 3 are internally provided with a first conductive medium 6. The first conductive medium 6 includes rectangular elongated conductive media 61 and folded conductive media 62. The size and shape of the conductive media are set to achieve different effects and cause the conductive media to be ductile and stable. In an embodiment which is not part of the scope of the present invention, the conductive contacts 4 can be connected by means of a conductive fabric belt 7. The conductive fabric belt 7 can connect conductive wires at different locations by the methods of single-point abutting, swapping interconnection, overlapping, bifurcating interconnection, convergent interconnection and cornering. The upper layer of the conductive fabric belt 7 is an insulating fabric waterproof layer, the middle is provided with a second conductive medium 8 (second conductive medium 8 not being part of the claimed subject-matter), the lower layer is a stretchable adhesive-coated waterproof layer, and the second conductive medium 8 is provided with a plurality of conductive nodes 9. Different circuits can be formed through interconnection of a plurality of conductive fabric belts. The second conductive medium 8 includes rectangular elongated conductive media 61 and folded conductive media 62.

There are a plurality of the folded conductive media 62 with a wavelength of L and an amplitude of M, and a rectangular elongated conductive medium 61 with a length of 2M and a width of M is arranged on the central axis position at each interval of N sawtooth waves.

In the foregoing technical solution, the first conductive medium 6 and the second conductive medium 8 (second conductive medium 8 not being part of the claimed subject-matter) typically are conductive metals, conductive cables, conductive inks or conductive adhesives formed by conductive particles. The conductive particles refer to carbon black, graphene, graphite, silver and metal powders, including conductive nanoparticles, etc. The lamination methods with a single conductive point can be soldering, bonding, stitching, etc.

The conductive wires 3 are connected to the zipper body 2, one side of the first layer of the zipper body is a reinforcing core 10, and the other side is a zipper fabric belt 12 with a marking position 11. The second layer is a plurality of parallel striped conductive media 13 with a width of K and perpendicular to the reinforcing core 10. The distance between the center points of two adjacent parallel striped conductive media 13 is W. The third layer is an insulating waterproof layer covering the parallel striped conductive media 13. The insulating waterproof layer is provided with a marking position 11, exposes a separate conductive medium with a width of K, and is electrically connected to a metal clamping portion built in the zipper body.

In the foregoing technical solution, the reinforcing core 10 is used for strengthening the firmness of the occlusion between the circuit fabric 1 and the zipper teeth 2 for higher durability and tolerance to force. The marking position 11 facilitates the connection of the conductive wires 3. By setting a distance between the center points of two adjacent parallel striped conductive media 13, the two parallel striped conductive media 13 maintain a certain distance, avoiding confusion of wires.

The zipper body 2 is provided with a first tooth 14 and a second tooth 15, which are distributed left and right in a stagger manner. The upper end of the first tooth 14 is provided with a placement slot 16, the lower end is provided with a placement block 17, and the placement slots 16 and placement blocks 17 on different sides form a structure of left-right staggered buckling. The middle of the second tooth 15 is provided with a metal probe 18, the middle of the first tooth 14 is provided with a connecting slot 19, and the metal probe 18 and the connecting slot 19 are both electrically connected to the parallel striped conductive media 13.

In the foregoing technical solution, the first tooth 14 on a side can be engaged with the first tooth 14 on the other side so that the zipper body 2 is closed, forming a closed circuit. When the first tooth 14 on a side is engaged with the first tooth 14 on the other side, the placement slot 16 is connected to the placement block 17, improving the vertical tensile strength of the zipper body. The second tooth 15 is fit with the first tooth 14, causing the metal probe 18 at the head of the second tooth 15 to be placed into and electrically connected to the connecting slot 19 of the first tooth 14.

Preferably, outside of the second tooth 15 is an elastic resin zipper tooth, the metal probe 18 is a V-shaped metal clip 20. The rear end of the V-shaped metal clip 20 has a first clamping portion 21. The first clamping portion 21 clamps the parallel striped conductive media 13. Outside of the first tooth 14 is a rigid resin zipper tooth. The connecting slot 19 is an H-shaped metal clip 22.The rear end of the H-shaped metal clip 22 has a second clamping portion 23. The second clamping portion 23 clamps the parallel striped conductive media 13.

In the foregoing technical solution, the first tooth 14 and the second tooth 15 are molded through injection molding, and the elastic resin zipper tooth of the second tooth 15 is closely engaged with the rigid resin zipper tooth of the first tooth 14 to enhance the waterproofness of the zipper body and to maintain the flexibility of the zipper body 2 as well. The V-shaped metal clip 20 and the H-shaped metal clip 22 are arranged inside the second tooth 15 and the first tooth 14. Through the wrapping of the first tooth 14 and the second tooth 15, the external surface of the zipper 2 is under insulating waterproof treatment to avoid electric leakage or short circuit. The first clamping portion 21 and the second clamping portion 23 ensure that the V-shaped metal clip 20 and the H-shaped metal clip 22 form a stable electrical connection with the parallel striped conductive media 13 and after the zipper 2 is closed, the electrical conduction is stable.

Preferably, the second tooth 15, which is made of resin for its external part is a plastic-steel zipper tooth. The metal probe 18 is a V-shaped metal clip 20. The rear end of the V-shaped metal clip 20 has a first clamping portion 21. The first clamping portion 21 clamps the parallel striped conductive media 31. The first tooth 14, which is made of resin for its external part is a plastic-steel zipper tooth. The connecting slot 19 is a Y-shaped metal clip 24. The rear end of the Y-shaped metal clip 24 has a third clamping portion 25. The third clamping portion 25 clamps the parallel striped conductive media 13.

In the foregoing technical solution, the first tooth 14 and the second tooth 15 are molded through injection molding, and the elastic resin zipper tooth of the second tooth 15 is closely engaged with the rigid resin zipper tooth of the first tooth 14 to enhance the waterproofness of the zipper body and to maintain the flexibility of the zipper body 2 as well. The V-shaped metal clip 20 and the Y-shaped metal clip 24 are arranged inside the second tooth 15 and the first tooth 14. Through the wrapping of the first tooth 14 and the second tooth 15, the external surface of the zipper 2 is under insulating waterproof treatment to avoid electric leakage or short circuit. The first clamping portion 21 and the second clamping portion 23 ensure that the V-shaped metal clip 20 and the Y-shaped metal clip 24 form a stable electrical connection with the parallel striped conductive media 13 and after the zipper 2 is closed, the electrical conduction is stable.

Preferably, the first conductive medium 6 is made of 316L stainless steel metal fiber sewing threads. Not being part of the claimed subject-matter, the second conductive medium 8 is made of 316L stainless steel metal fiber sewing threads. The first conductive medium 6 and the second conductive medium 8 (which is not part of the scope of the present invention) form rectangular elongated conductive media 61 and folded conductive media 62 by the zigzag stitching method.

In the foregoing technical solution, 316L is widely used in the chemical industry because of its excellent corrosion resistance. 316L is also a stainless-steel derivative of 18-8 austenitic stainless steel, with 2 to 3% element Mo added. On the basis of 316L, many steel grades have been derived, such as 316Ti by adding a small amount of Ti, 316N by adding a small amount of N, and 317L by increasing the content of Ni and Mo. Using 316L stainless steel metal fiber as sewing threads causes the first conductive medium 6 and the second conductive medium 8 (second conductive medium 8 not being part of the claimed subject-matter) to have good electrical conductivity and corrosion resistance. In daily use, the conductive fabric body 1 can be washed with water and does not come off easily.

Preferably, the first conductive medium 6 and the second conductive medium 8 (second conductive medium 8 not being part of the claimed subject-matter) are made of resilient conductive adhesive and directly laminated on the insulating fabric waterproof layer 5, and the lower layer is a liftable adhesive waterproof coating.

In the foregoing technical solution, the resilient conductive adhesive adopts 60-80% conductive particles, 20-30% adhesive, and 5-10% gelling agent. The conductive adhesive can be laminated directly to the bottom of the insulating fabric waterproof layer 5, forming stretchable elongated conductive media. The lamination method can be soldering, bonding or sewing, etc. The lower layer is a stretchable adhesive coating.

Preferably, the clothes body 27 is made through tailoring of the conductive fabric body 1, detachable glove bodies 28 are arranged at the cuffs of the smart clothes body 27, and the zipper teeth on a side of a zipper body 2 arranged at the sleeve of a glove body 28 are electrically connected to the zipper teeth on the other side of the zipper body 2 arranged at the clothes body 27. A plurality of flexible sensors are arranged at the joints of the clothes body 27 and the glove bodies 28, and electrically connected to the zipper teeth on a side of the zipper body 2 by means of the conductive wires 3 of the conductive fabric body 1, and the zipper teeth on the other side of the zipper body 2 are electrically connected to external electronic devices by means of the conductive wires 3 of the conductive fabric body 1.

In the foregoing technical solution, a plurality of conductive wires 3 are arranged inside the clothes body 27, forming different circuits. The glove bodies 28 can be electrically connected to the clothes body 27, achieving data transmission. Through the clothes body 27, the glove bodies 28, the zipper body 2 and the flexible sensors, the clothes body 27 can sense activities of the human body and achieve to report electrical signals to a controller. By electrically connecting a plurality of conductive wires 3 arranged inside a pocket to different devices, the functions like body movement analysis, health monitoring and man-machine interaction can be achieved.

Preferably, the zipper body at each cuff of the clothes body 27 can also be electrically connected to a flexible screen. The flexible screen establishes an electrical connection with the zipper teeth on the two sides of the zipper body 2 by means of the conductive fabric body 1, and a Velcro sticker is arranged on the back of the flexible screen. During use, the flexible screen is laminated with the external surface of the cuff, and then the zipper is pulled to close the zipper body.

In the foregoing technical solution, the clothes body 27 can connect the flexible screen and cause the flexible screen to be attached to a cuff of the clothes body 27, achieving data transmission between the flexible screen and the clothes body 27.

The flexible material for flexible sensors is a concept opposite to a rigid material. Generally, flexible materials have properties such as softness, low modulus and easy deformation. Common flexible materials include: polyvinyl alcohol (PVA), polyester (PET), paper sheets and textile material, etc. Flexible sensors are sensors made of flexible materials, have good flexibility and ductility, can be freely bent or even folded, adopt flexible and diverse structural forms and can be freely arranged according to the requirements of the measurement conditions to achieve easy measurement and detection. New types of flexible sensors are widely used in electronic skin, healthcare, electronic engineering, sports equipment, textiles, aeronautics and astronautics, environmental monitoring and other fields.

Flexible sensors include flexible pressure sensors, flexible gas sensors, flexible humidity sensors, flexible temperature sensors, flexible strain sensors, flexible magnetic impedance sensors and flexible heat flow sensors. According to the sensing mechanisms, flexible sensors include flexible resistive sensors, flexible capacitive sensors, flexible piezomagnetic sensors and flexible inductive sensors.

The flexible sensors are flexible capacitive sensors or flexible resistive sensors.

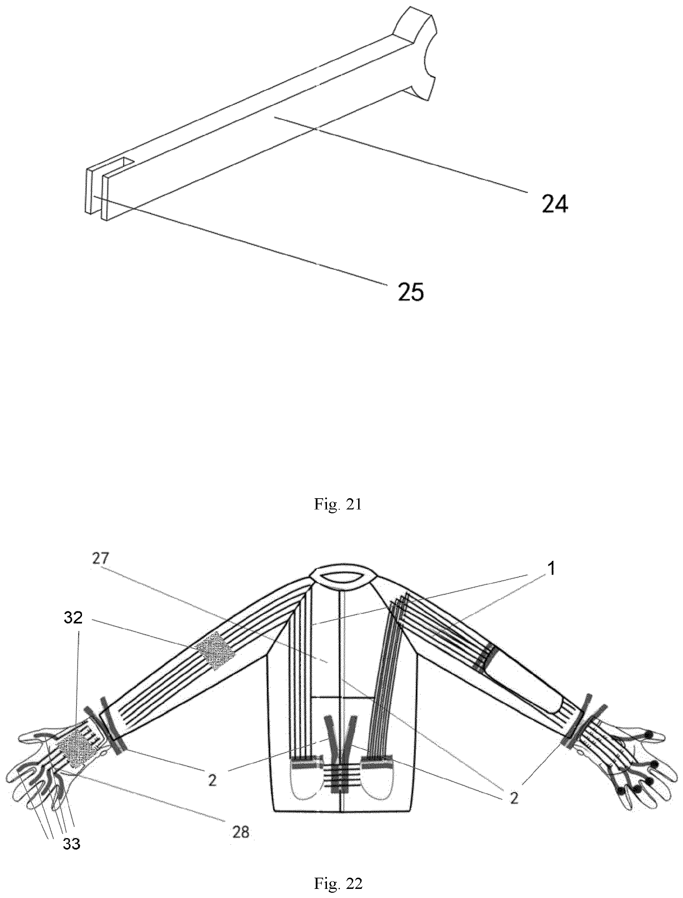

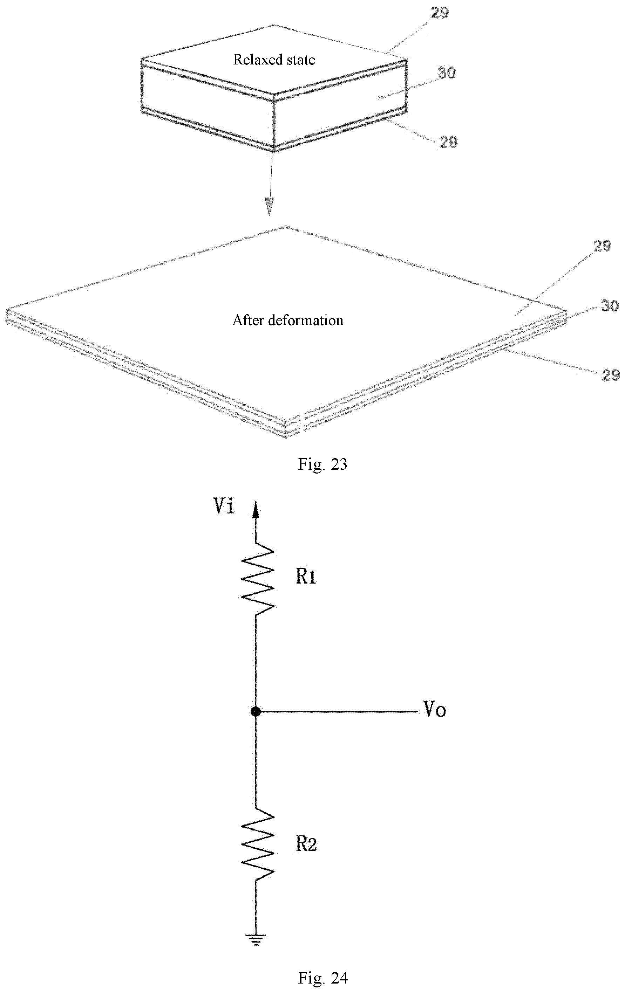

By referring to Fig. 22 and Fig. 23, the flexible sensors in the present invention are flexible capacitive sensors 32. The conductive electrode layer 29 is a conductive layer. The flexible organosilicon film 30 is a flexible organosilicon film made of electroactive polymer (not electrically conductive).

In the foregoing technical solution, the flexible capacitive sensors are suitable for monitoring pressure over large human body area, such as center of palm, and back, etc. Each flexible capacitive sensor is a parallel-plate capacitor consisting of electrodes and a dielectric layer, with a capacitance value C=E*R*A/d, where E is a vacuum dielectric constant, R is a relative dielectric constant, A is an effective area of electrode, and d is distance between electrode plates. When the capacitor is made of a soft material, the three variables of the capacitance value are liable to be affected by pressure. When the pressure increases, the dielectric layer gets thinner, the effective area of the electrodes gets larger, and the distance between the electrode plates gets smaller. Change in capacitance can be detected in the process of deformation under the condition of a voltage applied. Therefore, the pressure can be measured based on the change of capacitance signal, and the pressure at the center of palm or on the back can be monitored, thereby determining the posture of the human body, such as propping up on the ground, and lying down.

By referring to Fig. 22 and Fig. 24, alternatively, the flexible sensors in the present invention can be flexible resistive sensors 33.

In the foregoing technical solution, flexible resistive sensors are suitable for monitoring the bending displacement of small parts of the body, such as fingers, elbows and wrists. In the flexible resistive sensors, the distance between conductive particles is very short. When a sensor is kept straight, the resistance is low. But when it is bent, the distance between the particles increases, resulting in an increase in resistance and a decrease in current. The formula for output voltage Vo is Vo=Vi(R1/(R1+R2)), where Vi is input voltage, 5V in general, R1 is the resistance value of the flexible resistive element, and R2 is the resistance value of the fixed resistor. When the resistive element is bent, change will occur in the resistance value R1. When the input voltage is fixed, the output voltage will change according to the degree of bending of the resistive element, so that the bending activities of the human body parts such as fingers, elbows and wrists can be monitored, thereby determining movements such as gripping and hand turning.

Preferably, the conductive fabric body 1 and the zipper fabric belt 12 are formed integrally and sprayed on both sides with resilient insulating waterproof organosilicon gel.

In the foregoing technical solution, the insulating waterproof organosilicon gel has the same characteristics as other silicone, such as high temperature resistance, UV resistance, insulation and hydrophobicity, and can effectively isolate moisture. Further, the internal circuit components can be wrapped with waterproof breathable films to better protect the circuit.

Preferably, the zipper body 2 is a waterproof zipper body, and the internals of the elastic resin zipper teeth and the rigid resin zipper teeth are highly closely engaged and waterproof.

In the foregoing technical solution, when the smart clothes need to be washed, the electronic device can be removed through the zipper, avoiding the problem that water enters the electronic device during wash of the clothes body.

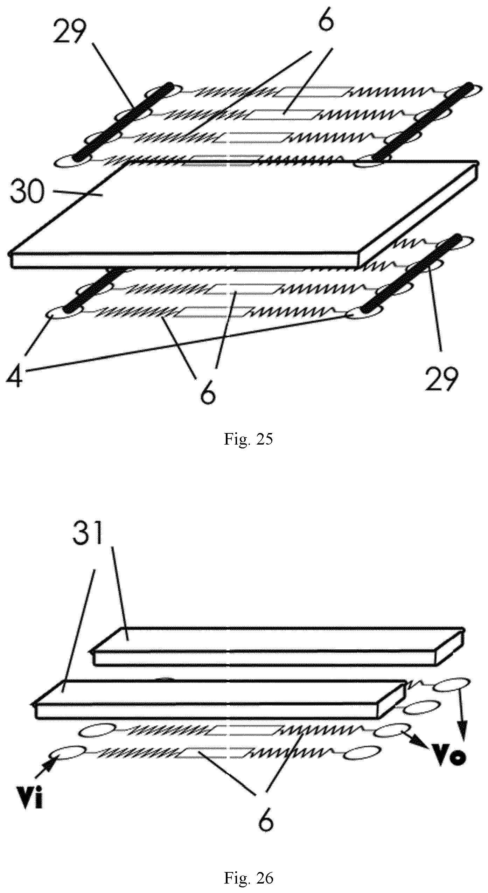

By referring to Fig. 25, the flexible capacitive sensors 32 have the following structure: in two square conductive fabric bodies 1, a plurality of the conductive contacts 4 of the first conductive medium 6 are electrically connected in parallel to form an upper conductive electrode layer 29 and a lower conductive electrode layer 29 relative to the middle dielectric layer, which is made of an organosilicon material. The middle flexible organosilicon film 30 and the upper and lower first conductive medium 6 layers jointly constitute a flexible capacitor, which can absorb and store electric charges. When being squeezed under a pressure, the conductive fabric body 1 and the flexible organosilicon film 30 both become thinner and wider together, and the capacitance changes.

By referring to Fig. 26, the flexible resistive sensors 33 have the following structure, of the two conductive wires 3 adjacent to the conductive fabric body 1, one is a voltage input terminal, and the other is a voltage output terminal. A graphite coated silicone layer 31 with certain conductivity is arranged over the first conductive medium 6. The graphite coated silicone layer 31 as a flexible resistive element is flexible and stretchable. When the conductive fabric body 1 and the graphite coated silicone layer 31 are bent together, the output voltage will change.

Working principle: The clothes body 27 is made through tailoring of the conductive fabric body 1. A plurality of conductive wires 3 and conductive contacts 4 are arranged on the conductive fabric body 1, the middle of the clothes body 27 is provided with a zipper body 2, zipper bodies 2 are arranged at cuffs and pockets, flexible sensors are arranged inside the clothes body 27, i.e., the joints of human body, and flexible sensors are arranged inside the glove bodies 28, i.e., the joints of palms to sense movements of the human body.

During use, after the clothes body 27 is worn, one side of the zipper body arranged at the sleeve of each glove body 28 is connected to the other side of the zipper body 2 arranged at a cuff of the clothes body 27 for closure, thereby achieving data inter-communication between the glove bodies 28 and the clothes body 27. When the zipper body 2 is closed, the metal probe 18 and the connecting slot 19 are engaged with each other so that the V-shaped metal clip 20 is placed into the H-shaped metal clip 22 or the Y-shaped metal clip 24, the left and right sides of the clothes body 27 constitute a circuit, and electrical signals corresponding to movements of the human body are transmitted through flexible sensors to a microprocessor, thereby identifying movements of the human body, sending data to the cloud/a controller and achieving different feedbacks.

The foregoing embodiments only represent the preferred embodiments of the present invention. Their descriptions are concrete and detailed, but they shall not be therefore understood as limitations to the scope of the present invention patent. Therefore, the protection scope of the present invention patent should be based on the appended claims.

## Claims

1. A zipper-type circuit fabric, comprising a conductive fabric body (1) and a zipper body (2), wherein a plurality of conductive wires (3) and conductive contacts (4) are distributed inside the conductive fabric body (1), a plurality of conductive contacts (4) being arranged on two sides of the exterior of the zipper body (2), an end of the conductive fabric body (1) being connected to one side of the zipper body (2), the plurality of conductive wires (3) and conductive contacts (4) being able to electrically connected to a plurality of electronic components, a stretchable adhesive coating being arranged on each of the two sides of the conductive wires (3), the conductive wires (3) being internally provided with a first conductive medium (6), the first conductive medium (6) including rectangular elongated conductive media (61) and folded conductive media (62),
**characterized in that**, the conductive fabric body (1) is provided with an insulating fabric waterproof layer (5), the plurality of conductive contacts (4) and the plurality of conductive wires (3) being covered by the insulating fabric waterproof layer (5),
wherein there are a plurality of the folded conductive media (62) with a wavelength of L and an amplitude of M, and a rectangular elongated conductive medium (61) with a length of 2M and a width of M being arranged on the central axis position at each interval of N sawtooth waves,
wherein the conductive wires (3) are connected to the zipper body (2), one side of the first layer of the zipper body (2) being a reinforcing core (10), the other side being a zipper fabric belt (12) with a marking position (11), the second layer being a plurality of parallel striped conductive media (13) with a width of K and perpendicular to the reinforcing core (10), the distance between the center points of two adjacent parallel striped conductive media (13) being W, the third layer being an insulating layer covering the parallel striped conductive media (13), and the insulating layer exposing a separate conductive medium with a width of K and the marking position (11) and being laminated/sewn with other conductive wires (3),
the zipper body (2) is provided with a first tooth (14) and a second tooth (15), which are distributed left and right in a stagger manner, the upper end of the first tooth (14) being provided with a placement slot (16), the lower end being provided with a placement block (17), the placement slots (16) and the placement blocks (17) on different sides forming a structure of left-right staggered buckling, the middle of the second tooth (15) being provided with a metal probe (18), the middle of the first tooth (14) being provided with a connecting slot (19), and the metal probe (18) and the connecting slot (19) both being electrically connected to the conductive wires (3).

2. The zipper-type circuit fabric according to claim 1, wherein outside of the second tooth (15) is an elastic resin zipper tooth, the metal probe (18) being a V-shaped metal clip (20), the rear end of the V-shaped metal clip (20) having a first clamping portion (21), the first clamping portion (21) clamping the parallel striped conductive media (13), outside of the first tooth (14) being a rigid resin zipper tooth, the inner connecting slot (19) being an H-shaped metal clip (22), the rear end of the H-shaped metal clip (22) having a second clamping portion (23), and the second clamping portion (23) clamping the parallel striped conductive media (13).

3. The zipper-type circuit fabric according to claim 1, wherein outside of the second tooth (15) is an elastic resin zipper tooth, the metal probe (18) being a V-shaped metal clip (20), the rear end of the V-shaped metal clip (20) having a first clamping portion (21), the first clamping portion (21) clamping the parallel striped conductive media (13), outside of the first tooth (14) being a rigid resin zipper tooth, the connecting slot (19) being a Y-shaped metal clip (24), the rear end of the Y-shaped metal clip (24) having a third clamping portion (25), and the third clamping portion (25) clamping the parallel striped conductive media (13).

4. The zipper-type circuit fabric according to claim 2 or 3, wherein the first conductive medium (6) is made of 316L stainless steel metal fiber sewing threads, and the first conductive medium (6) forming rectangular elongated conductive media (61) and folded conductive media (62) by the zigzag stitching method.

5. The zipper-type circuit fabric according to claim 2 or 3, wherein the first conductive medium (6) is made of resilient conductive adhesive and directly laminated on the insulating fabric waterproof layer (5), the lamination method being soldering, bonding or sewing, and the lower layer being a liftable adhesive coating.

6. A smart clothes composed of the zipper-type circuit fabric as in claim 5, comprising a clothes body (27) and a zipper body (2), wherein the clothes body (27) is made through tailoring of the conductive fabric body (1), detachable glove bodies (28) being arranged at the cuffs of the clothes body (27), a plurality of conductive contacts (4) being arranged at the sleeves of the glove bodies (28), the conductive contacts (4) being connected to the conductive wires (3), the front of the clothes body (27) being provided with a zipper body (2), a plurality of flexible sensors being arranged at the joints of the clothes body (27) and the glove bodies (28), the flexible sensors being electrically connected to the conductive wires (3), and a plurality of the conductive contacts (4) distributed in the pockets being electrically connected to external electronic devices.

7. The smart clothes according to claim 6, wherein a plurality of conductive contacts (4) are arranged at the cuffs of the clothes body (27), and cooperated with a plurality of conductive contacts (4) which being arranged at the sleeves of the glove bodies (28), a plurality of conductive contacts (4) being arranged at the cuffs of the clothes body (27) and being able to be electrically connected to a flexible screen by means of the zipper teeth, and a Velcro sticker being arranged on the back of the flexible screen and being able to be laminated on the surface of a cuff.

8. The smart clothes according to claim 6, wherein the flexible sensors are flexible capacitive sensors (32) or flexible resistive sensors (33).

9. The smart clothes according to claim 7, wherein the conductive fabric body (1) and the zipper fabric belt (12) are formed integrally and sprayed on both sides with resilient insulating waterproof organosilicon gel.

## Patentansprüche

1. - Reißverschlussartiges Schaltungsgewebe, umfassend einen leitenden Gewebekörper (1) und einen Reißverschlusskörper (2), wobei innerhalb des leitenden Gewebekörpers (1) eine Vielzahl von leitenden Drähten (3) und leitenden Kontakten (4) verteilt sind, eine Vielzahl von leitenden Kontakten (4) auf zwei Seiten der Außenseite des Reißverschlusskörpers (2) angeordnet sind, ein Ende des leitenden Gewebekörpers (1) mit einer Seite des Reißverschlusskörpers (2) verbunden ist, die Vielzahl von leitenden Drähten (3) und leitenden Kontakten (4) in der Lage sind, elektrisch mit einer Vielzahl von elektronischen Komponenten verbunden zu werden, eine dehnbare Klebebeschichtung auf jeder der zwei Seiten der leitenden Drähte (3) angeordnet ist, die leitenden Drähte (3) intern mit einem ersten leitenden Medium (6) versehen sind, das erste leitende Medium (6) rechteckige, längliche leitende Medien (61) und gefaltete leitende Medien (62) umfasst,
**dadurch gekennzeichnet, dass** der leitende Gewebekörper (1) mit einer isolierenden, wasserdichten Gewebeschicht (5) versehen ist, die Vielzahl von leitenden Kontakten (4) und die Vielzahl von leitenden Drähten (3) von der isolierenden, wasserdichten Gewebeschicht (5) bedeckt sind,
wobei es eine Vielzahl der gefalteten leitenden Medien (62) mit einer Wellenlänge L und einer Amplitude M gibt, und ein rechteckiges längliches leitendes Medium (61) mit einer Länge von 2M und einer Breite von M an der mittleren Achsposition in jedem Intervall von N Sägezahnwellen angeordnet ist,
wobei die leitenden Drähte (3) mit dem Reißverschlusskörper (2) verbunden sind, eine Seite der ersten Schicht des Reißverschlusskörpers (2) ein Verstärkungskern (10) ist, die andere Seite ein Reißverschlussgewebeband (12) mit einer Markierungsposition (11) ist, die zweite Schicht eine Vielzahl von parallelen, streifenförmigen leitenden Medien (13) mit einer Breite K und senkrecht zu dem verstärkenden Kern (10) ist, wobei der Abstand zwischen den Mittelpunkten von zwei benachbarten, parallelen, streifenförmigen leitenden Medien (13) W ist, die dritte Schicht eine Isolierschicht ist, die das parallele, streifenförmige leitende Medium (13) bedeckt, und die Isolierschicht ein separates leitendes Medium mit einer Breite K und der Markierungsposition (11) freilegt und mit anderen leitenden Drähten (3) laminiert/vernäht ist,
der Reißverschlusskörper (2) mit einem ersten Zahn (14) und einem zweiten Zahn (15) versehen ist, die links und rechts versetzt verteilt sind, das obere Ende des ersten Zahns (14) mit einem Einsetzschlitz (16) versehen ist, das untere Ende mit einem Einsetzblock (17) versehen ist, die Einsetzschlitze (16) und die Einsetzblöcke (17) auf verschiedenen Seiten eine links-rechts versetzte Knickstruktur bilden, die Mitte des zweiten Zahns (15) mit einer Metallsonde (18) versehen ist, die Mitte des ersten Zahns (14) mit einem Verbindungsschlitz (19) versehen ist, und die Metallsonde (18) und der Verbindungsschlitz (19) beide elektrisch mit den leitenden Drähten (3) verbunden sind.

2. - Reißverschlussartiges Schaltungsgewebe nach Anspruch 1, wobei eine Außenseite des zweiten Zahns (15) ein elastischer Kunststoff-Reißverschlusszahn ist und die Metallsonde (18) eine V-förmige Metallklammer (20) ist, wobei das hintere Ende der V-förmigen Metallklammer (20) einen ersten Klemmabschnitt (21) aufweist, der erste Klemmabschnitt (21) das parallele streifenförmige leitende Medium (13) einklemmt, eine Außenseite des ersten Zahns (14) ein starrer Kunststoff-Reißverschlusszahn ist, der innere Verbindungsschlitz (19) eine H-förmige Metallklammer (22) ist, das hintere Ende der H-förmigen Metallklammer (22) einen zweiten Klemmabschnitt (23) aufweist, und der zweite Klemmabschnitt (23) das parallele streifenförmige leitende Medium (13) einklemmt.

3. - Reißverschlussartiges Schaltungsgewebe nach Anspruch 1, wobei eine Außenseite des zweiten Zahns (15) ein elastischer Kunststoff-Reißverschlusszahn ist, die Metallsonde (18) eine V-förmige Metallklammer (20) ist, wobei das hintere Ende der V-förmigen Metallklammer (20) einen ersten Klemmabschnitt (21) aufweist, der erste Klemmabschnitt (21) das parallele streifenförmige leitende Medium (13) einklemmt, eine Außenseite des ersten Zahns (14) ein starrer Kunststoff-Reißverschlusszahn ist, der Verbindungsschlitz (19) eine Y-förmige Metallklammer (24) ist, wobei das hintere Ende der Y-förmigen Metallklammer (24) einen dritten Klemmabschnitt (25) aufweist, und der dritte Klemmabschnitt (25) das parallele streifenförmige leitende Medium (13) einklemmt.

4. - Reißverschlussartiges Schaltungsgewebe nach Anspruch 2 oder 3, wobei das erste leitende Medium (6) aus Metallfasernähfäden aus Edelstahl 316L gefertigt ist, und das erste leitende Medium (6) rechteckige längliche leitende Medien (61) und gefaltete leitende Medien (62) durch das Zickzack-Nähverfahren bildet.

5. - Reißverschlussartiges Schaltungsgewebe nach Anspruch 2 oder 3, wobei das erste leitende Medium (6) aus elastischem, leitendem Klebstoff gefertigt ist und direkt auf die isolierende, wasserdichte Gewebeschicht (5) laminiert ist, das Laminierungsverfahren Löten, Kleben oder Nähen ist, und die untere Schicht eine abziehbare Klebebeschichtung ist.

6. - Intelligente Kleidung, bestehend aus dem reißverschlussartigen Schaltungsgewebe nach Anspruch 5, umfassend einen Kleidungskörper (27) und einen Reißverschlusskörper (2), wobei der Kleidungskörper (27) durch Zuschneiden des leitenden Gewebekörpers (1) gefertigt wird, abnehmbare Handschuhkörper (28) an den Manschetten des Kleidungskörpers (27) angeordnet sind, eine Vielzahl von leitenden Kontakten (4) an den Stulpen der Handschuhkörper (28) angeordnet sind, wobei die leitenden Kontakte (4) mit den leitenden Drähten (3) verbunden sind, die Vorderseite des Kleidungskörpers (27) mit einem Reißverschlusskörper (2) versehen ist, eine Vielzahl von flexiblen Sensoren an den Verbindungsstellen des Kleidungskörpers (27) und der Handschuhkörper (28) angeordnet sind, wobei die flexiblen Sensoren elektrisch mit den leitenden Drähten (3) verbunden sind, und eine Vielzahl der in den Taschen verteilten leitenden Kontakte (4) elektrisch mit externen elektronischen Vorrichtungen verbunden sind.

7. - Intelligente Kleidung nach Anspruch 6, wobei eine Vielzahl von leitenden Kontakten (4) an den Manschetten des Kleidungskörpers (27) angeordnet sind und mit einer Vielzahl von leitenden Kontakten (4) zusammenwirken, die an den Stulpen der Handschuhkörper (28) angeordnet sind, eine Vielzahl von leitenden Kontakten (4), die an den Manschetten des Kleidungskörpers (27) angeordnet sind und in der Lage sind, mittels der Reißverschlusszähne elektrisch mit einem flexiblen Schirm verbunden zu werden, und ein Klettaufkleber auf der Rückseite des flexiblen Schirms angeordnet ist und in der Lage ist, auf die Oberfläche einer Manschette laminiert zu werden.

8. - Intelligente Kleidung nach Anspruch 6, wobei die flexiblen Sensoren flexible kapazitive Sensoren (32) oder flexible resistive Sensoren (33) sind.

9. - Intelligente Kleidung nach Anspruch 7, wobei der leitende Gewebekörper (1) und das Reißverschlussgewebeband (12) einstückig gebildet und beidseitig mit elastischem, isolierendem, wasserdichtem Organosiliciumgel besprüht sind.

## Revendications

1. - Tissu de circuit de type fermeture à glissière, comprenant un corps de tissu conducteur (1) et un corps de fermeture à glissière (2), dans lequel une pluralité de fils conducteurs (3) et de contacts conducteurs (4) sont répartis à l'intérieur du corps de tissu conducteur (1), une pluralité de contacts conducteurs (4) étant disposés sur deux côtés de l'extérieur du corps de fermeture à glissière (2), une extrémité du corps de tissu conducteur (1) étant reliée à un côté du corps de fermeture à glissière (2), les différents fils conducteurs (3) et de contacts conducteurs (4) étant aptes à être connectés électriquement à une pluralité de composants électroniques, un revêtement adhésif extensible étant disposé sur chacun des deux côtés des fils conducteurs (3), les fils conducteurs (3) comportant intérieurement un premier support conducteur (6), le premier support conducteur (6) comprenant un support conducteur rectangulaire allongé (61) et un support conducteur plié (62),
**caractérisé par le fait que** le corps de tissu conducteur (1) comporte une couche imperméable de tissu isolant (5), les différents contacts conducteurs (4) et les différents fils conducteurs (3) étant couverts par la couche imperméable de tissu isolant (5),
dans lequel il y a une pluralité de milieux conducteurs pliés (62) avec une longueur d'onde de L et une amplitude de M, et un milieu conducteur rectangulaire allongé (61) avec une longueur de 2M et une largeur de M étant disposé sur la position d'axe central à chaque intervalle de N ondes en dents de scie,
dans lequel les fils conducteurs (3) sont reliés au corps de fermeture à glissière (2), un côté de la première couche du corps de fermeture à glissière (2) étant un noyau de renforcement (10), l'autre côté étant une ceinture de tissu de fermeture à glissière (12) avec une position de marquage (11), la deuxième couche étant une pluralité de supports conducteurs à bandes parallèles (13) d'une largeur de K et perpendiculaires au noyau de renforcement (10), la distance entre les points centraux de deux supports conducteurs à bandes parallèles adjacentes (13) étant W, la troisième couche étant une couche isolante recouvrant les supports conducteurs à bandes parallèles (13), et la couche isolante exposant un support conducteur séparé d'une largeur de K et la position de marquage (11) et étant laminée/cousue avec d'autres fils conducteurs (3),
le corps de fermeture à glissière (2) comporte une première dent (14) et une deuxième dent (15), qui sont réparties à gauche et à droite en quinconce, l'extrémité supérieure de la première dent (14) comportant une fente de placement (16), l'extrémité inférieure comportant un bloc de placement (17), les fentes de placement (16) et les blocs de placement (17) sur différents côtés formant une structure d'emboîtage élastique en quinconce gauche-droite, le milieu de la deuxième dent (15) comportant une sonde métallique (18), le milieu de la première dent (14) comportant une fente de connexion (19), et la sonde métallique (18) et la fente de connexion (19) étant toutes deux connectées électriquement aux fils conducteurs (3).

2. - Tissu de circuit de type fermeture à glissière selon la revendication 1, dans lequel l'extérieur de la deuxième dent (15) est une dent de fermeture à glissière en résine élastique, la sonde métallique (18) étant une pince métallique en forme de V (20), l'extrémité arrière de la pince métallique en forme de V (20) ayant une première partie de serrage (21), la première partie de serrage (21) serrant le support conducteur à bandes parallèles (13), à l'extérieur de la première dent (14) se trouvant une dent de fermeture à glissière en résine rigide, la fente de connexion interne (19) étant une pince métallique en forme de H (22), l'extrémité arrière de la pince métallique en forme de H (22) ayant une deuxième partie de serrage (23), et la deuxième partie de serrage (23) serrant le support conducteur à bandes parallèles (13).

3. - Tissu de circuit de type fermeture à glissière selon la revendication 1, dans lequel l'extérieur de la deuxième dent (15) est une dent de fermeture éclair en résine élastique, la sonde métallique (18) étant une pince métallique en forme de V (20), l'extrémité arrière de la pince métallique en forme de V (20) ayant une première partie de serrage (21), la première partie de serrage (21) serrant le support conducteur à bandes parallèles (13), l'extérieur de la première dent (14) étant une dent de fermeture à glissière en résine rigide, la fente de connexion (19) étant une pince métallique en forme de Y (24), l'extrémité arrière de la pince métallique en forme de Y (24) ayant une troisième partie de serrage (25), et la troisième partie de serrage (25) serrant le support conducteur à bandes parallèles (13).

4. - Tissu de circuit de type fermeture à glissière selon la revendication 2 ou 3, dans lequel le premier support conducteur (6) est fait de fils de couture en fibre métallique en acier inoxydable 316L, et le premier support conducteur (6) formant un support conducteur rectangulaire allongé (61) et un support conducteur plié (62) par la méthode de piqûre en zigzag.

5. - Tissu de circuit de type fermeture à glissière selon la revendication 2 ou 3, dans lequel le premier support conducteur (6) est fait d'un adhésif conducteur élastique et directement laminé sur la couche imperméable de tissu isolant (5), la méthode de laminage étant le soudage, le collage ou la couture, et la couche inférieure étant un revêtement adhésif soulevable.

6. - Vêtement intelligent composé d'un tissu à circuit de type fermeture à glissière selon la revendication 5, comprenant un corps de vêtement (27) et un corps de fermeture à glissière (2), dans lequel le corps de vêtement (27) est fabriqué par la confection du corps de tissu conducteur (1), des corps de gants détachables (28) étant disposés aux poignets du corps de vêtement (27), une pluralité de contacts conducteurs (4) étant disposés aux manches des corps de gants (28), les contacts conducteurs (4) étant connectés aux fils conducteurs (3), l'avant du corps du vêtement (27) comportant un corps de fermeture à glissière (2), plusieurs capteurs flexibles étant disposés aux articulations du corps du vêtement (27) et des corps de gants (28), les capteurs souples étant connectés électriquement aux fils conducteurs (3), et une pluralité des contacts conducteurs (4) répartis dans les poches étant connectés électriquement à des dispositifs électroniques externes.

7. - Vêtement intelligent selon la revendication 6, dans lequel plusieurs contacts conducteurs (4) sont disposés aux poignets du corps de vêtement (27), et coopèrent avec une pluralité de contacts conducteurs (4) qui sont disposés aux manches des corps de gants (28), plusieurs contacts conducteurs (4) étant disposés aux poignets du corps de vêtement (27) et étant aptes à être connectés électriquement à un écran souple au moyen des dents de fermeture à glissière, et un autocollant à boucles et crochets étant disposé au dos de l'écran souple et étant apte à être laminé sur la surface d'un poignet.

8. - Vêtement intelligent selon la revendication 6, dans lequel les capteurs souples sont des capteurs capacitifs souples (32) ou des capteurs résistifs souples (33).

9. - Vêtement intelligent selon la revendication 7 , dans lequel le corps en tissu conducteur (1) et la ceinture en tissu de fermeture à glissière (12) sont formés d'un seul tenant et pulvérisés des deux côtés avec un gel organosiliconé résistant, isolant et imperméable.
